# EUROPEAN PATENT SPECIFICATION

(11) **EP 1 558 212 B1**
(45) Date of publication and mention of the grant of the patent: **11.08.2010**
(21) Application number: 03809723.4
(22) Date of filing: 07.10.2003
(51) Int. Cl.: A61K 9/00, A61K 8/00

(54) **PHARMACEUTICAL AND COSMETIC FORMULATIONS**
PHARMAZEUTISCHE UND KOSMETISCHE ZUBEREITUNGEN
FORMULATIONS PHARMACEUTIQUES ET COSMETIQUES

(30) Priority: 31.10.2002 DE 10250711
(43) Date of publication of application: 03.08.2005
(73) Proprietor: Evonik Degussa GmbH, 45128 Essen (DE)
(72) Inventor: HASENZAHL, Steffen, Morris Plains, NJ 07950 (US); DRECHSLER, Margarete, 63571 Gelnhausen (DE)
(86) International application number: PCT/EP2003/011054
(87) International publication number: WO 2004/039349

(56) References cited:
- EP-A- 0 637 616
- US-A- 4 034 077
- US-A- 5 776 240

## Description

The invention relates to pharmaceutical and cosmetic formulations which comprise hydrophobic highly disperse silicon dioxide.

In a medicament a distinction is made between two substance groups with different functions, namely active compounds and auxiliary substances.

The active compounds are characterized by their specific pharmacological action. They are the active constituent of a medicament. As such, they are also identified quantitatively on the packaging and on the pack leaflet.

In addition to the actual active compound, medicaments comprise auxiliary substances or also adjuvants in order to convert the active compound into suitable formulations which are active at the desired site of use. A medicament conventionally comprises several auxiliary substances with different functions, for example fillers, binders, disintegrating agents, lubricants, greasing agents or mould release agents.

A large number of auxiliary substances can be resorted to in the development of stable, easy-to-handle and active medicaments from active compound(s) and auxiliary substances.

Highly disperse silicon dioxide, such as, for example, Aerosil^{®} 200, is an important auxiliary substance which is often employed for pharmaceutical and cosmetic formulations.

Highly disperse silicon dioxide is prepared by flame hydrolysis of chlorosilanes and is therefore also called pyrogenic silicon dioxide. It is listed in numerous pharmacopoeias as follows: "Hochdisperses Siliciumdioxid" (German Pharmacopoeia); "Silica, Colloidal Anhydrous" (European Pharmacopoeia); "Colloidal Silicon Dioxide" (US Pharmacopoeia / National Formulary), "Colloidal Anhydrous Silica" (British Pharmacopoeia) and "Light Anhydrous Silicic Acid" (Japanese Pharmacopoeia). Highly disperse silicon dioxide can be used, for example, in solid product forms as a flow regulating agent, adsorbent and desiccant and in liquid and semi-solid product forms as a suspension stabilizer and matrix- and gel-forming agent. It can furthermore be used to increase the mechanical stability and the rate of disintegration of tablets. It can moreover improve the distribution of the active compound. In a few medicaments highly disperse silicon dioxide also functions as the active compound.

Highly disperse, pyrogenic silicon dioxide has a high affinity for water and is wetted completely by this. It is distinguished by hydrophilic properties.

Hydrophobic highly disperse silicon dioxide, such as, for example, Aerosil^{®} R 972, can have significant advantages over hydrophilic highly disperse silicon dioxide in pharmaceutical and cosmetic compositions. Although it is not described in the Pharmacopoeia, it has therefore been used by some pharmaceutical companies for many years. The Red List - the list of medical preparations for Germany - thus names a number of preparations in which Aerosil^{®} R 972 or methylated silicon dioxide is mentioned as an auxiliary substance.

Hydrophobic highly disperse silicon dioxide as a pharmaceutical raw material is described generally by H. P. Fiedler, Lexikon der Hilfsstoffe [Dictionary of Auxiliary Substances], Editio Cantor Verlag, Aulendorf. Aerosil^{®} R 812 and R 972 are dealt with explicitly here. Information on the use of Aerosil^{®} R 972 in pharmaceutical and cosmetic compositions is moreover to be found in the publication series Pigmente Nr. 49, Aerosil in Pharmazie und Kosmetik [Pigments no. 49, Aerosil in Pharmacy and Cosmetics], Degussa.

Hydrophobic highly disperse silicon dioxide, such as, for example, Aerosil^{®} R 972, is suitable as a flow regulator for hygroscopic, pulverulent substances. By the formation of a layer of Aerosil^{®} R 972 particles on the powder particles, the water (vapour) uptake thereof is reduced or slowed down (H.P. Fiedler, Lexikon der Hilfsstoffe [Dictionary of Auxiliary Substances], Editio Cantor Verlag Aulendorf, 3rd edition, 1989). Furthermore, no film of water forms on the particles of the hydrophobic silicon dioxide itself, so that the adhesive forces between the "coated" powder particles remain low. In this manner, for example, an addition of 0.5 wt.% Aerosil^{®} R 972 acquires the flow properties of maize starch even at high atmospheric humidities (H. v. Czetsch Lindenwald et al., J. Soc. Cosmetics Chemists 16 (1965) 251). On the other hand, if Aerosil^{®} 200, which is hydrophilic, is used, lumping together of hygroscopic substances - even with relatively high Aerosil contents - often cannot be prevented. Hygroscopic powders contained in capsules also remain flowable by an addition of Aerosil^{®} R 972, which is hydrophobic (H.P. Fiedler, Lexikon der Hilfsstoffe [Dictionary of Auxiliary Substances], Editio Cantor Verlag Aulendorf, 3rd edition, 1989).

Aerosil^{®} R 972 can also be employed in the granulation of hygroscopic products, for example plant extracts. This is even possible from aqueous solutions, so that organic solvents can be omitted. Hydrophilic silicon dioxide is unsuitable here.

Aerosil^{®} R 972 moreover improves the properties of powder raw materials. Thus, for example, the scatter value of kieselguhr is increased eight-fold (F. Gstirner, Arch. Pharmz. 300 (1967) 757). Powders moreover retain their consistency, even at high relative atmospheric humidities.

There are also advantages in tablet-making from hygroscopic powders or granules. Hydrophobic highly disperse silicon dioxide is superior here if a slow tablet disintegration or a delayed release of active compound, for example in the case of sustained release formulations, is to be achieved. Hydrophilic silicon dioxide accelerates tablet disintegration in many cases, since it can be wetted by water and promotes the transportation of water into the inside of the tablet in this manner (wick effect). Together with water-swellable compounds, it is therefore also employed as a disintegrating agent. Since hydrophobic silicon dioxide is not wetted by water, it shows no wick effect.

Some specific examples of sustained-release formulations for solid, oral medicament forms with Aerosil^{®} R 972 are described in the following:

In ibuprofen tablets Aerosil^{®} R 972 reduces the release of active compound to a greater degree than hydrophilic highly disperse silicon dioxides (E.M. Samy et al.; Bull. Pharm. Sci. Assiut University 19 (1996) 19).

If acetaminophen or theophylline is subjected to dry granulation with Aerosil^{®} R 972 and the resulting mixture is introduced into capsules, the active compound release rate thereof is reduced drastically. An addition of 0.6 wt.% Aerosil^{®} R 972 is optimum. With this, 80 - 100% of the active compound is released within eight hours (V.R. Sista et al.; Drug Development and Industrial Pharmacy, 22 (1996) 153).

Aspartate tablets or mineral salt-containing gelatine capsules with a slow release of active compound can be prepared using Aerosil^{®} R 972 (O. Gattnar, Slovakian Patent CS 236300, 1985, L. Gyarmati et al., Hungarian Patent HU 26263, 1983). Capsules with a delayed release of active compound are also described by Takeda Chem. Ind. Ltd., Japanese Patent 0 823 9301, 1996. These contain a "network" of water-soluble carboxymethylcellulose and polyvalent salts, in which is enclosed the active compound dissolved in water. According to the patent specification, Aerosil^{®} R 972 serves as an adsorbent.

Aerosil^{®} R 972 is moreover the most effective flow auxiliary in hard gelatine capsule fillings (H.v. Czetsch-Lindenwald et al., J. Soc. Cosmetics Chemists 16 (1965) 251).

Hydrophilic highly disperse silicon dioxide is unsuitable for stabilizing or thickening w/o emulsions, since it migrates into the aqueous phase because of its hydrophilic character (H.v. Czetsch-Lindenwald, Pharm. Ind. 27 (1965) 300). In contrast, stabilization is effected with Aerosil^{®} R 972, because this remains in the oily phase as hydrophobic material and builds up a gel structure here. W/o ointments formulated with Aerosil^{®} R 972 thus still remain spreadable 10 to 20ºC above their melting point. The release of aqueous active compounds from such bases is furthermore slowed down.

Aerosil^{®} R 972 thickens balsam gels to a considerably lower degree than hydrophilic highly disperse silicon dioxides. This is advantageous if highly disperse silicon dioxide is employed as an active compound carrier or for conversion of paste-like active compounds into pulverulent ones (E. Toricht et al., Pharmazie 32 (1977) 109).

3% Aerosil^{®} R 972 is sufficient for the preparation of 10% ZnO suspensions in oils, while larger amounts of hydrophilic highly disperse silicon dioxide are required in order to achieve the same effect. After storage for 100 days, according to H.v. Czetsch-Lindenwald, Pharm. Ind. 27 (1965) 300, gels form, which can easily be liquefied again by shaking. The content of Aerosil^{®} R 972 is not noticed on the skin.

Highly disperse silicon dioxide is a valuable auxiliary substance in the preparation of suppositories: It prevents the sedimentation of suspended active compounds during pouring and solidification by increasing the viscosity of the molten base, influences - for example in eutectic mixtures - the melting properties and the breaking strength of the products, and can be used as a carrier for incorporation of liquid auxiliary substances. Here also hydrophobic highly disperse silicon dioxide has advantages over the hydrophilic variant in a number of uses (H. Rupprecht et al., Deutsche Apotheker Zeitung 11 (1978) 385).

Thus, the viscosity of molten hard fat which contains 4 wt.% aminophenazone is increased considerably by 4 wt.% Aerosil^{®} R 972, while the effect of 4 wt.% hydrophilic highly disperse silicon dioxide is low (H. Rupprecht et al., Deutsche Apotheker Zeitung 11 (1978) 385). A uniform distribution of the active compound in the suppository mass can be ensured more easily with Aerosil^{®} R 972 in this manner than with hydrophilic highly disperse silicon dioxide. The former moreover slows down the release of the active compound to a greater degree than the latter (H. Rupprecht et al. Pharmazie 32 (1977) 354). The delayed release of a water-soluble active compound from a Witepsol W 35 suppository mass prepared with 2% Aerosil^{®} R 972 is described in H.v. Czetsch-Lindenwald, Pharm. Ind. 27 (1965) 300. Suppositories with sustained release of active compound which comprise the water-soluble active compound morphine sulfate, a swellable organic compound (hydroxypropylmethylcellulose) and Aerosil^{®} R 972 are described by T. Jauw, European Patent 550 100 B1, 1996.

Medical patches, the adhesive layer of which comprises in each case 7.1 wt.% of Aerosil^{®} R 972 and hydrophilic highly disperse silicon dioxide (based on the dry matter), in addition to the active compound and various polymers, are described by Sekisui Chem. Ind. Com. Ltd., *Japanese Patent* 0 625 6178, 1996 and Sekisui Chem. Ind. Com. Ltd., *Japanese Patent* 0 625 6173, 1994 and *Japan. Patent* 0 431 2525, 1992). Aerosil^{®} R 972 and hydrophilic highly disperse silicon dioxide increase the viscosity of the solution containing polymer and active compound which is applied to the support and dried. The active compounds are optionally also adsorbed on to the surface of the highly disperse silicon dioxide, the consequence of which is a slower and more uniform release of the active compound.

Aerosil^{®} R 972 and R 812 are furthermore employed for the preparation of pharmaceutical and cosmetic formulations bottled in pressurized gas bottles (H. v. Czetsch Lindenwald et al., J. Soc. Cosmetics Chemists 16 (1965) 251).

Injection solutions based on Aerosil^{®} R 974-containing w/o emulsions are described, for example, in EP 1 179 349 A1.

Since the highest purity requirements must be met in the preparation of pharmaceutical and cosmetic products, the considerable development of dust in particular presents problems when working with hydrophobic highly disperse silicon dioxide types - commercially available products are, for example, Aerosil^{®} R 972 and Aerosil^{®} R 974 (both Degussa), Wacker HDK H15 and Wacker HDK H20 (both Wacker) and Cab-O-Sil TS 610 and Cab-O-Sil TS 620 (both Cabot). Since hydrophobic highly disperse silicon dioxide types as a rule have finer particles than the hydrophilic products (e.g. Aerosil^{®} 200), the dust problem is even more serious here. Another disadvantage is the low bulk and tamped density of the hydrophobic product types, typical values are 40-50 g/l, which causes a considerable additional expenditure on labour and time in the preparation of pharmaceutical and cosmetic formulations.

In the use of hydrophobic highly disperse silicon dioxide in pharmaceutical and cosmetic formulations, an improved flowability of mixtures produced with this would furthermore be desirable, in order to be able to achieve, for example, a higher dosing accuracy in the production of tablets and capsules. By this means, it would be possible on the one hand to achieve a lower variation in tablet and capsule weights and on the other hand to improve the profitability of processes which lead to these presentation forms.

The object of the present invention is to provide pharmaceutical and cosmetic formulations which avoid the disadvantages of the prior art.

The invention provides pharmaceutical and cosmetic formulations which comprise hydrophobic highly disperse silicon dioxide, which are **characterized in that** the silicon dioxide has a tamped density of 70 to 400 g/l, determined in accordance with DIN 55943.

The invention also provides pharmaceutical and cosmetic formulations which comprise hydrophobic highly disperse silicon dioxide, which are **characterized in that** the silicon dioxide contains a maximum of 3.0 wt.% of water-wettable contents.

The invention also provides pharmaceutical and cosmetic formulations which comprise hydrophobic highly disperse silicon dioxide, which are **characterized in that** the silicon dioxide has a tamped density of 70 to 400 g/l, determined in accordance with DIN 55943, and contains a maximum of 3.0 wt.% of water-wettable contents.

It has been found that when working with the formulations according to the invention only a low development of dust occurs and the flowability of the formulations is significantly higher than in the case of those according to the prior art. In addition, the mechanical stability of tablets is improved and the capsule weight is increased. Furthermore, the release properties of tablets and capsules can be adjusted in a controlled manner.

This result is surprising, since it was not possible to assume that the properties, such as, for example, flowability or mechanical stability, of the pharmaceutical and cosmetic formulations are influenced by the tamped density of the pyrogenic silicon dioxide used. According to the article "Kolloidale Kieselsaure als Gelbildner [Colloidal silica as a gel-forming agent]" (www.pharmazeutische-zeitung.de/pza/2001-51/pharm.5.htm) it was even to be expected that compacted highly disperse silicas have disadvantages compared with the non-compacted product types. Problems are described here with Aerosil^{®} 200 V (tamped density 120 g/l), since it does not achieve the required thickening performance compared with the standard products Aerosil^{®} 200 (tamped density 50 g/l).

It is furthermore surprising that the release of active compounds and the disintegration time of the pharmaceutical and cosmetic formulations is influenced by the tamped density of the hydrophobic silicon dioxide used.

It has been found that it is particularly favourable to choose a tamped density of the hydrophobic highly disperse silicon dioxide of between 70 and 400 g/l, in particular between 75 and 300 g/l.

It is furthermore advantageous to choose hydrophobic highly disperse silicon dioxide with a BET surface area, determined in accordance with DIN 66131, of 50 to 400 m²/g. A BET surface area of 90-300 m²/g is particularly advantageous.

The preparation of the silicon dioxide is known, for example, from Ullmann's Encyclopedia of Industrial Chemistry, vol. A23, page 635 et seq., 5th edition, 1993.

Hydrophilic highly disperse silicon dioxide can be prepared by flame hydrolysis of chlorosilanes and is very pure chemically. It carries silanol groups on its surface. As a result it has a high affinity for water - it is hydrophilic - and is wetted completely by this. Alkyl groups can be anchored chemically on the surface of the substance by reaction of the silanol groups with organic silicon compounds. The resulting products are then no longer wetted by water, they are hydrophobic.

Aerosil^{®} R 972 and Aerosil^{®} R 974 are thus formed by reacting freshly prepared Aerosil^{®} with dimethyldichlorosilane in an inert gas atmosphere at 400 to 600ºC in the presence of water vapour (publication series Pigmente Nr. 5, "Hydrophobes Aerosil, Herstellung, Eigenschaften und Anwendungen" [Pigments no. 5, "Hydrophobic Aerosil, Preparation, Properties and Uses"], Degussa). Aerosil can also be partly or completely hydrophobized with other organosilanes. Examples of these are Aerosil^{®} R 812 (reaction with hexmethyldisilazane), Aerosil^{®} R 805 (reaction with trimethoxyoctylsilane) and Aerosil^{®} R 202 (with silicone oil). Processes for treatment with a surface-modifying agent are to be found, for example, in DE-A-11 63 784, DE-A-196 16 781, DE-A-197 57 210 or DE-A-44 02 370.

The hydrophobic highly disperse silicon dioxide acquires its tamped density either directly during the preparation or in a subsequent process step. Thus, for example, compacting processes for pyrogenic silicon dioxide are described in DE-A-32 38 427 and DE-A-37 41 846. The high tamped density can furthermore be achieved by a grinding such as is described, for example, in EP 0 637 616 A1. Granules of hydrophobic highly disperse silicon dioxide from EP 0 725 037 also have a high tamped density and are suitable according to the invention for pharmaceutical and cosmetic formulations.

Hydrophobic highly disperse silicon dioxide types which are suitable according to the invention and are already commercially available are Aerosil^{®} R 972 V, Aerosil^{®} R 974 V, Aerosil^{®} R 976 V (Degussa), Aerosil^{®} R 8200 (Degussa), Aerosil^{®} R 972 W (Nippon Aerosil Corporation), Wacker HDK H15P, HDK H2000 and HDK H3004 (Wacker) and Reolosil DM10 (Tokuyama). Aerosil^{®} R 972 V, Aerosil^{®} R 974 V and Aerosil^{®} R 972 W, and compacted Aerosil^{®} R 812 and Aerosil^{®} 812 S are particularly suitable.

Hydrophobic highly disperse silicon dioxide is not wetted by water. Various methods are known for determination of the hydrophobicity or the degree of hydrophobization, for example the methanol wettability of Corning Glass.

A simple method for determination of the water-wettable contents is described in the following: About 0.2 g of substance, weighed accurately to 0.001 g, are shaken intensively with 50 ml of water in a 250 ml pear-shaped separating funnel for 1 min. The funnel is then left to stand for one hour. During this, the predominant portion of the solid floats up. Without shaking up the suspension again, 45 ml of the liquid, which may be slightly cloudy, are drained off dropwise and transferred to a dish which has been dried at 140ºC and cooled in a desiccator. The liquid is evaporated off completely at 110 - 150ºC, during which it should be ensured that no substance sprays out. After cooling in a desiccator, the dish is weighed again. The weight difference with respect to the empty dish should be not more than 0.006 g. This corresponds to 3.0 wt.% of the substance weighed out. Hydrophobic highly disperse silicon dioxide in which the water-wettable contents make up a max. of 3.0 wt.% are particularly suitable for the pharmaceutical and cosmetic formulations according to the invention.

Pyrogenic silicon dioxide also includes doped oxides and mixed oxides in which the silicon dioxide content is at least 90%. Doped pyrogenic silicon dioxides can be obtained, for example, by the process described in DE-A-196 50 500, in which the doping is introduced via an aerosol of a salt solution or suspension in a flame such as is used for the preparation of pyrogenic oxides. A mixed oxide with a silicon dioxide content of greater than 90 wt.% can be obtained, for example, by the process described in DE-A-199 19 635.

Mixtures of pyrogenic silicon dioxide with doped silicon dioxide with an SiO₂ content of 90%, with mixed oxides with an SiO₂ content of 90% or more and/or hydrophobized silicon dioxide can also be used for the formulations according to the invention.

The hydrophobic highly disperse silicon dioxide is preferably present in the formulation according to the invention to the extent of 0.01 to 30 wt.%, particularly preferably to the extent of 0.1 to 15.0 wt.%. It is conventionally employed as an auxiliary substance, but can also be used as an active compound, the action then primarily being a physical action.

Hydrophobic highly disperse silicon dioxide with a tamped density of between 70 and 400 g/l can be employed according to the invention in any desired solid, semi-solid or liquid pharmaceutical formulations (medicament forms), preferably for oral and/or topical uses, for example in suspensions, emulsions, aerosols, injection solutions, ointments, creams, gels, pastes, suppositories, sticks, powders, dusting powders, granules, tablets, pastilles, coated tables, film-coated tablets, hard gelatine capsules, soft gelatine capsules, extrudates, microcapsules or microspherules. Solid medicament forms, such as, for example, powders, dusting powders, granules, tablets and capsules, are particularly preferred.

The term pharmaceutical formulations in the context of the present invention also includes precursors and intermediates products for the preparation of granules, tablets, capsules, suspensions, inspissated juices and inspissated drops. Such precursors and intermediate products can have e.g. the form of a powder, granules or an extrudate.

Methods for the preparation of solid, semi-solid and liquid medicament forms are known and are described in numerous publications and textbooks of pharmaceutical technology, cf. for example K.H. Bauer, K.-H. Frömming, C. Führer, Lehrbuch der pharmazeutischen Technologie [Textbook of Pharmaceutical Technology], 6th edition, Wissenschaftliche Verlagsgesellschaft mbH Stuttgart 1999.

The formulations according to the invention can comprise any desired pharmaceutical active compound. Examples which may be mentioned are: α-proteinase inhibitor, abacavir, abciximab, acarbose, acetylsalicylic acid, acyclovir, adenosine, albuterol, aldesleukin, alendronate, alfuzosin, alosetron, alprazolam, alteplase, ambroxol, amifostine, amiodarone, amisulpride, amlodipine, amoxicillin, amphetamine, amphotericin, ampicillin, amprenavir, anagrelide, anastrozole, ancrod, anti-haemophilia factor, aprotinin, atenolol, atorvastatin, atropine, azelastine, azithromycin, azulene, barnidipine, beclomethasone, benazepril, benserazide, beraprost, betamethason, betaxolol, bezafibrate, bicalutamide, bisabolol, bisoprolol, botulinus toxin, brimonidine, bromazepam, bromocriptine, budesonide, bupivacaine, bupropion, buspirone, butorphanol, cabergoline, calcipotriene, calcitonin, calcitriol, camphor, candesartan, candesartan cilexetil, captopril, carbamazepine, carbidopa, carboplatin, carvedilol, cefaclor, cefadroxil, cefaxitin, cefazolin, cefdinir, cefepime, cefixime, cefmetazole, cefoperazone, cefotiam, cefoxopran, cefpodoxime, cefprozil, ceftazidime, ceftibuten, ceftriaxone, cefuroxime, celecoxib, celiprolol, cephalexin, cerivastatin, cetirizine, chloramophenicol, cilastatin, cilazapril, cimetidine, ciprofibrate, ciprofloxacin, cisapride, cisplatin, citalopram, clarithromycin, clavulanic acid, clindamycin, clomipramine, clonazepam, clonidine, clopidogrel, clotrimazole, clozapine, cromolyn, cyclophosphamide, cyclosporin, cyproterone, dalteparin, deferoxamine, desogestrel, dextroamphetamine, diazepam, diclofenac, didanosine, digitoxin, digoxin, dihydroergotamine, diltiazem, diphtheria protein, diphtheria toxoxide, divalproex, dobutamine, docetaxel, dolasetron, donepezil, dornase-α, dorzolamide, doxazosin, doxifluridine, doxorubicin, dydrogesterone, ecabet, efavirenz, enalapril, enoxaparin, eperison, epinastine, epirubicin, eptifibatide, erythropoietin-α, erythropoietin-β, etanercept, ethinyloestradiol, etodolac, etoposide, factor VIII, famciclovir, famotidine, faropenem, felodipine, fenofibrate, fenoldopam, fentanyl, fexofenadine, filgrastim, finasteride, flomoxef, fluconazole, fludarabine, flunisolide, flunitrazepam, fluoxetine, flutamide, fluticasone, fluvastatin, fluvoxamine, follitropin-α, follitropin-β, formoterol, fosinopril, furosemide, gabapentin, gadodiamide, ganciclovir, gatifloxacin, gemcitabin, gestodene, glatiramer, glibenclamide, glimepiride, glipizide, glyburide, goserelin, granisetron, griseofulvin, hepatitis B antigen, hyaluronic acid, hycosin, hydrochlorothiazide, hydrocodone, hydrocortisone, hydromorphone, hydroxychloroquine, hylan g-f 20, ibuprofen, ifosfamide, imidapril, imiglucerase, imipenem, immunoglobulin, indinavir, indomethacin, infliximab, insulin, insulin, human, insulin lispro, insulin aspart, interferon-β, interferon-α, iodine-125, iodixanol, iohexol, iomeprol, iopromide, iopromide, ioversol, ioxoprolen, ipratropium, ipriflavone, irbesartan, irinotecan, isosorbide, isotretinoin, isradipine, itraconazole, potassium chlorazepate, potassium chloride, ketorolac, ketotifen, whooping-cough vaccine, coagulation factor IX, lamivudine, lamotrigin, lansoprazole, latanoprost, leflunomide, lenograstim, letrozole, leuprolide, levodopa, levofloxacin, levonorgestrel, levothyroxine, lidocaine, linezolid, lisinopril, lopamidol, loracarbef, loratadine, lorazepam, losartan, lovastatin, lysine-acetylsalicylic acid, manidipin, mecobalamin, medroxyprogesterone, megestrol, meloxicam, menatetrenone, meningococcus vaccine, menotropin, meropenem, mesalamine, metaxalone, metformin, methylphenidate, methylprednisolone, metoprolol, midazolam, milrinone, minocycline, mirtazapine, misoprostol, mitoxantrone, moclobemide, modafinil, mometasone, montelukast, morniflumate, morphium, moxifloxacin, mycophenolate, nabumetone, nadroparin, naproxen, naratriptan, nefazodone, nelfinavir, nevirapine, niacin, nicardipine, nicergoline, nifedipine, nilutamide, nilvadipine, nimodipine, nitroglycerine, nizatidine, norethyndron, norfloxacin, octreotid, olanzapin, omeprazole, ondansetron, orlistat, oseltamivir, oestradiol, oestrogens, oxaliplatin, oxaprozin, oxolinic acid, oxybutynin, paclitaxel, palivizumab, pamidronate, pancrelipase, panipenem, pantoprazole, pantoprazole, paracetamol, paroxetine, pentoxifylline, pergolide, phenytoin, pioglitazon, piperacillin, piroxicam, pramipexole, pravastatin, prazosin, probucol, progesterone, propafenone, propofol, propoxyphen, prostaglandin, quetiapine, quinapril, rabeprazole, raloxifene, ramipril, ranitidine, repaglinide, reserpine, ribavirin, riluzole, risperidone, ritonavir, rituximab, rivastigmine, rizatriptan, rofecoxib, ropinirole, rosiglitazon, salmeterol, saquinavir, sargramostim, serrapeptase, sertraline, sevelamer, sibutramine, sildenafil, simvastatin, somatropin, somatropin, sotalol, spironolactone, stavudine, sulbactam, sulfaethidole, sulfamethoxazole, sulfasalazine, sulpiride, sumatriptan, tacrolimus, tamoxifen, tamsulosin, tazobactam, teicoplanin, temocapril, temozolomide, tenecteplase, tenoxicam, teprenone, terazosin, terbinafine, terbutaline, tetanus toxoid, tetrabenazine, tetrazapam, thymol, tiagabine, tibolone, ticarcillin, ticlopidine, timolol, tirofiban, tizanidine, tobramycin, tocopheryl nicotinate, tolterodine, topiramate, topotecan, torasemide, tramadol, trandolapril, trastuzumab, triamcinolone, triazolam, trimebutine, trimethoprim, troglitazone, tropisetron, tulobuterol, unoprostone, urofollitropin, valacyclovir, valproic acid, valsartan, vancomycin, venlafaxine, verapamil, verteporfin, vigabatrin, vinorelbine, vinpocetine, voglibose, warfarin, zafirlukast, zaleplon, zanamivir, zidovudine, zolmitriptan, zolpidem, zopiclone and derivatives thereof. However, pharmaceutical active compounds are also to be understood as meaning other substances, such as vitamins, provitamins, essential fatty acids, extracts of plant and animal origin and oils of plant and animal origin.

The pharmaceutical compositions in which hydrophobic highly disperse silicon dioxide with a tamped density of between 70 and 400 g/l can be employed also include plant medicament formulations and homoeopathic formulations.

The pharmaceutical formulations according to the invention can also be so-called sustained release and depot medicament forms with controlled release of the active compounds. The pharmaceutical formulations according to the invention can furthermore also be part of therapeutic systems, such as, for example, therapeutic systems for local use and transdermal therapeutic systems.

Further constituents of the pharmaceutical compositions can be conventional auxiliary substances, such as, for example, antioxidants, binders, emulsifiers, dyestuffs, film-forming agents, fillers, aroma substances, flavourings, gel-forming agents, preservatives, solvents, oils, powder bases, ointment bases, acids and salts for recipe formulation, small-scale preparation and preparation of pharmaceutical compositions, greasing agents, disintegrating agents, suppository bases, suspension stabilizers, sweeteners, propellant gases, plasticizers and sugar substitutes.

According to an advantageous embodiment, the formulations according to the invention can comprise as the active compound paracetamol, acetylsalicylic acid or ibuprofen.

The hydrophobic highly disperse silicon dioxide with a tamped density of between 70 and 400 g/l can furthermore be used according to the invention in cosmetic formulations of any desired consistency, for example in powders, liquids, foams, sprays, gels, creams, ointments, pastes, sticks or tablets. The cosmetic formulations can accordingly be single- or multi-phase systems, such as, for example, emulsions, suspensions or aerosols.

The cosmetic formulation according to the invention can be, for example, a soap; a syndet; a liquid washing or shower preparation; a bath additive; a make-up removal composition; a peeling preparation; a skin cream; a skin lotion; a face mask; a foot care composition; a sunscreen composition; a skin tanning composition; a depigmenting composition; an insect-repellent composition; a wet shaving composition, such as, for example, a stick, a cream, a gel or a foam; a pre-shave preparation; an after-shave care composition; a hair removal composition; a dental cream; a hair shampoo; a hair care composition, such as, for example, a hair treatment course, a rinse or a conditioner; a permanent wave composition; a straightening composition, a style setting composition, such as, for example, a hair setting composition, a hair spray, a hair lacquer, a hair gel or a hair wax; a hair colour-modifying composition, such as, for example, a blonding composition, a hair-colouring composition, a toner or a colour enhancer; a deodorant or an antiperspirant composition, such as, for example, a stick, a roll-on, a lotion, a powder or a spray; a face make-up, such as, for example, a tinted day cream, a powder cream, a face powder, a cream make-up or a rouge; an eye make-up, such as, for example, a lid shadow, a mascara, a kajal stick, an eyeliner or an eyebrow pencil; a lip care composition; a decorative lip care composition, such as, for example, a lipstick, a lip gloss or a lip contour pencil; or a nail care composition, such as, for example, a nail varnish, a nail varnish remover, a cuticle remover, a nail hardener or a nail care cream.

The present invention also provides a cosmetic formulation which comprises the hydrophobic highly disperse silicon dioxide and at least one constituent chosen from absorbents, astringents, antimicrobial substances, antioxidants, antiperspirants, antifoams, antidandruff active compounds, antistatics, binders biological additives, bleaching agents, chelating agents, deodorizing agents, emollients, emulsifiers, emulsion stabilizers, depilatory agents, dyestuffs, humectants, film-forming agents, aroma substances, flavourings, hair-colouring agents, preservatives, corrosion protection agents, cosmetic oils, solvents, oral care substances, oxidizing agents, plant constituents, buffer substances, reducing agents, abrasives, surfactants, propellant gases, opacifying agents, UV filters and absorbers, denaturants, viscosity regulators and vitamins.

### Examples

### Pharmaceutical formulations:

The pulverulent starting substances are weighed accurately to 0.01 g in the stated sequence and mixed manually in a glass bottle. This mixture is sieved through a sieve of mesh width 0.71 mm and homogenized in a glass bottle with a Turbula mixer for five minutes.

**Table 1: Formulations (data in wt.%)**

| | Formulation 1 | Formulation 2 | Formulation 3 |
|---|---|---|---|
| Paracetamol | 83.3 | - | - |
| Acetylsalicylic acid | - | 83.3 | - |
| Lactose | - | | 79.7 |
| Powdered cellulose | 13.3 | 10.4 | 20.0 |
| Maize starch | 3.0 | 5.0 | - |
| Magnesium stearate | 0.1 | - | - |
| Stearic acid | - | 1.0 | - |
| Silicon dioxide | 0.3 | 0.3 | 0.3 |

Aerosil^{®} R 972 (tamped density approx. 50 g/l; comparison examples) and Aerosil^{®} R 972 V (tamped density 90 g/l; according to the invention) are used as the silicon dioxide.

The flow rating and/or poured cone height are determined as a measure of the flowability. Furthermore, tablets are pressed and capsules filled with the formulations according to table 1.

### Hard gelatine capsules

Using a capsule filling apparatus, hard gelatine capsules of size 1 with an empty weight of 71 - 78 mg are filled with the formulations according to table 1. In each case 60 capsules are prepared and the average capsule weight is determined.

The values for formulation 1 are to be found in tab. 2, those for formulation 2 in tab. 3 and those for formulation 3 in tab. 4.

### Tablets

The formulations according to table 1 are pressed at the same pressing pressure using an eccentric press (EKO, Korsch) to give tablets with a weight of approx. 600 mg. The tablet hardness is determined on in each case 10 tablets by means of a semi-automatic hardness tester. The disintegration time in water warmed to 37ºC (manufacturer Erweka, model ZT 31) is moreover determined on six tablets.

The values for formulation 1 are to be found in tab. 2, those for formulation 2 in tab. 3 and those for formulation 3 in table 4.

**Table 2: Properties of formulation 1**

| Tamped density SiO₂ [g/l] | Poured cone height (cm) | Tablet hardness [N] | Disintegration time [s] | Capsule weight [mg] |
|---|---|---|---|---|
| 50 | 2.4 | 59 | 25 | 380 |
| 90 | 2.2 | 79 | 30 | 399 |

| | | | | |
|---|---|---|---|---|
| *Poured cone height determined in accordance with:Publication series Pigmente [Pigments], number 31 from Degussa, 6th edition. The lower the poured cone height of a powder mixture, the better the flow properties. | | | | |

**Table 3: Properties of formulation 2**

| Tamped density SiO₂ [g/l] | Poured cone height [cm] | Tablet hardness [N] | Disintegration time [s] | Capsule weight [mg] |
|---|---|---|---|---|
| 50 | 2.4 | 93 | 25 | 375 |
| 90 | 2.2 | 95 | 35 | 381 |

**Table 4: Properties of formulation 3**

| Tamped density SiO₂ [g/l] | Poured cone height [cm] | Tablet hardness [N] | Disintegration time [s] | Capsule weight [mg] |
|---|---|---|---|---|
| 50 | 2.3 | 141 | 25 | 345 |
| 90 | 2.2 | 238 | 75 | 350 |

The formulations according to the invention show clear advantages in flow properties, tablet hardness and capsule weight. They moreover have a longer disintegration time.

### Pharmaceutical auxiliary substance mixtures:

198.0 g Avicel PH101 and in each case 2.0 g Aerosil^{®} R 972 (Degussa; tamped density 50 g/l; comparison example), Aerosil^{®} R 972 V (Degussa; tamped density 90 g/l; according to the invention) and Aerosil^{®} R 972 W (Nippon Aerosil Corporation; tamped density 160 g/l) are premixed manually in a 1 l wide-necked bottle and the mixture is sieved through a 0.71 mm sieve and mixed in a free-fall mixer (Turbula) for 10 min at 42 revolutions per minute. The flow rating and poured cone height of the mixture were then determined.

The results of the experiments are summarized in table 5.

**Table 5: Properties of the pharmaceutical auxiliary substance mixtures**

| Tamped density SiO₂ [g/l] | Flow rating | Poured cone height [cm] |
|---|---|---|
| 50 | 3 | 2.0 |
| 90 | 2.5 | 1.9 |
| 160 | 2 | 1.75 |

| | | |
|---|---|---|
| *Flow rating and poured cone height determined in accordance with: Publication series Pigmente [Pigments], number 31 from Degussa, 6th edition. The lower the flow rating or poured cone height of a powder mixture, the better the flow properties. | | |

### Determination of the water-wettable contents of hydrophobic highly disperse silicon dioxide:

About 0.2 g of substance, weighed accurately to 0.001 g, are shaken intensively with 50 ml of water R in a 250 ml pear-shaped separating funnel for 1 min. The funnel is then left to stand for one hour. During this, the predominant portion of the solid floats up. Without shaking up the suspension again, 45 ml of the liquid, which may be slightly cloudy, are drained off dropwise and transferred to a dish which has been dried at 140ºC and cooled in a desiccator.
The liquid is evaporated off completely at 110 - 150ºC, during which it should be ensured that no substance sprays out. After cooling in a desiccator, the dish is weighed again. The weight difference with respect to the empty dish should be not more than 0.006 g. This corresponds to 3.0 wt.% of the substance weighed out.

### Table 6: Water-wettable contents of the hydrophobic highly disperse silicas used

| Product | Aerosil^{®} 972 | Aerosil^{®} 972 V | CP 1 | CP 2 |
|---|---|---|---|---|
| Tamped density (g/l) | 50 | 90 | 50 | 90 |
| Water-wettable contents (%) | 3.0 | 2.0 | 7.0 | 6.0 |

The comparison products CP 1 and CP 2 are prepared analogously to Aerosil^{®} R 972 and Aerosil^{®} R 972 V, but with a starting amount of dimethyldichlorosilane reduced by 10%. The products therefore have a somewhat higher content of water-wettable contents. Pharmaceutical formulations 2 and 3 from table 1 are also prepared with CP 1 and CP 2. The analytical data of the formulations are summarized in tables 7 and 8.

**Table 7: Properties of formulation 2**

| Product | Poured cone height [cm] | Tablet hardness [N] | Disintegration time [s] | Capsule weight [mg] |
|---|---|---|---|---|
| Aerosil ^{®} R 972 | 2.4 | 93 | 25 | 375 |
| CP 1 | 2.6 | 80 | 15 | 355 |
| Aerosil ^{®} R 972 V | 2.2 | 95 | 35 | 381 |
| CP 2 | 2.4 | 88 | 20 | 368 |

**Table 8: Properties of formulation 3**

| Product | Poured cone height [cm] | Tablet hardness [N] | Disintegration time [s] | Capsule weight [mg] |
|---|---|---|---|---|
| Aerosil ^{®} R 972 | 2.3 | 141 | 25 | 345 |
| CP 1 | 2.5 | 125 | 20 | 335 |
| Aerosil ^{®} R 972 V | 2.2 | 238 | 75 | 350 |
| CP 2 | 2.4 | 202 | 60 | 340 |

The experiments show that in addition to the tamped density, the water-wettable contents have a considerable influence on the properties of the pharmaceutical formulations. Hydrophobic highly disperse silicon dioxide in which the water-wettable contents make up a max. of 3.0 wt.% are accordingly particularly suitable for the pharmaceutical and cosmetic formulations according to the invention.

## Claims

1. Pharmaceutical and cosmetic formulations comprising hydrophobic highly disperse silicon dioxide,
**characterized in that**
the silicon dioxide has a tamped density of 70 to 400 g/l, determined in accordance with DIN 55943.

2. Formulations according to claim 1,
**characterized in that**
the BET surface area of the hydrophobic highly disperse silicon dioxide is between 50 and 400 m²/g.

3. Formulations according to claim 1 or 2,
**characterized in that**
the hydrophobic highly disperse silicon dioxide is present in the formulations to the extent of 0.01 to 30 wt.%.

4. Formulations according to claim 1 or 2 comprising hydrophobic highly disperse silicon dioxide, **characterized in that** the silicon dioxide contains a maximum of 3.0 wt.% of water-wettable contents.

5. Formulations according to claim 1 or 2 comprising hydrophobic highly disperse silicon dioxide, **characterized in that** the silicon dioxide has a tamped density of 70 to 400 g/l, determined in accordance with DIN 55943, and contains a maximum of 3.0 wt.% of water-wettable contents.

## Patentansprüche

1. Pharmazeutische und kosmetische Zubereitungen, enthaltend hydrophobes hochdisperses Siliciumdioxid, **dadurch gekennzeichnet, daß** das Siliciumdioxid eine Stampfdichte von 70 bis 400 g/l, bestimmt nach DIN 55943, aufweist.

2. Zubereitungen nach Anspruch 1, **dadurch gekennzeichnet, daß** die BET-Oberfläche des hydrophoben hochdispersen Siliciumdioxids zwischen 50 und 400 m²/g liegt.

3. Zubereitungen nach Anspruch 1 oder 2, **dadurch gekennzeichnet, daß** das hydrophobe hochdisperse Siliciumdioxid in den Zubereitungen in einer Menge von 0,01 bis 30 Gew.-% vorliegt.

4. Zubereitungen nach Anspruch 1 oder 2, enthaltend hydrophobes hochdisperses Siliciumdioxid, **dadurch gekennzeichnet, daß** das Siliciumdioxid maximal 3,0 Gew.-% wasserbenetzbare Anteile enthält.

5. Zubereitungen nach Anspruch 1 oder 2, enthaltend hydrophobes hochdisperses Siliciumdioxid, **dadurch gekennzeichnet, daß** das Siliciumdioxid eine Stampfdichte von 70 bis 400 g/l, bestimmt nach DIN 55943, aufweist und maximal 3,0 Gew.-% wasserbenetzbare Anteile enthält.

## Revendications

1. Formulations pharmaceutiques et cosmétiques comprenant du dioxyde de silicium hydrophobe hautement dispersé, **caractérisées en ce que** le dioxyde de silicium a une masse volumique tassée de 70 à 400 g/l, déterminée conformément à DIN 55943.

2. Formulations selon la revendication 1, **caractérisées en ce que** la surface BET du dioxyde de silicium hydrophobe hautement dispersé est comprise entre 50 et 400 m²/g.

3. Formulations selon la revendication 1 ou 2, **caractérisées en ce que** le dioxyde de silicium hydrophobe hautement dispersé est présent dans les formulations jusqu'à 0,01 à 30 % en poids.

4. Formulations selon la revendication 1 ou 2, comprenant du dioxyde de silicium hydrophobe hautement dispersé, **caractérisées en ce que** le dioxyde de silicium contient au maximum 3,0 % en poids de contenu mouillable à l'eau.

5. Formulations selon la revendication 1 ou 2, comprenant du dioxyde de silicium hydrophobe hautement dispersé, **caractérisées en ce que** le dioxyde de silicium a une masse volumique tassée de 70 à 400 g/l, déterminée conformément à DIN 55943, et contient au maximum 3,0 % en poids de contenu mouillable à l'eau.
